# EUROPEAN PATENT APPLICATION

(11) **EP 4 696 693 A1**
(43) Date of publication of application: **18.02.2026**
(21) Application number: 23937150.3
(22) Date of filing: 17.07.2023
(51) Int. Cl.: C07D 498/14, A61K 31/5383, A61P 31/16

(54) **HEXAHYDRO[1,4]OXAZINO[3,4-C]PYRIDO[2,1-F][1,2,4]TRIAZINE DEUTERATED DERIVATIVE, PHARMACEUTICAL COMPOSITION THEREOF, PREPARATION METHOD THEREFOR, AND ANTI-INFLUENZA USE THEREOF**

(30) Priority: 18.05.2023 CN 202310561285
(71) Applicant: Changsha Jingyi Pharmaceutical Technology Co., Ltd, Changsha, Hunan 410221 (CN)
(72) Inventor: ZHANG, Hailong, Changsha, Hunan 410221 (CN); DU, Le, Changsha, Hunan 410221 (CN); YU, Dan, Changsha, Hunan 410221 (CN)
(74) Representative: Plougmann Vingtoft a/s
(86) International application number: PCT/CN2023/107754
(87) International publication number: WO 2024/234474

(57) **Abstract**

The present application provides a novel compound and a preparation method therefor. The compound specifically relates to a cap-dependent nucleic acid PA endonuclease inhibitor and intermediates for preparation of the compound. Further provided are a pharmaceutical composition of the compound, and a use of the compound and the pharmaceutical composition of the compound in treating influenza. The compound is suitable for being prepared into drugs of various dosage forms, and can be widely used for treating seasonal influenza.

## Description

### TECHNICAL FIELD

The present application belongs to the technical field of medicines and, in particular, relates to a compound and a preparation method therefor, a pharmaceutical composition of the compound, and use of the compound and the pharmaceutical composition of the compound in the treatment of influenza.

### BACKGROUND

When the influenza virus enters a host cell, the replication and transcription within the host cell rely on the catalysis of RNA polymerase, and the cap-dependent nucleic acid PA endonuclease is an essential enzyme for the synthesis of viral mRNA. By inhibiting the cap-dependent nucleic acid PA endonuclease, the transcription process of the influenza virus can be selectively blocked, thereby achieving the goal of preventing, treating or alleviating influenza in patients.

Existing commonly used anti-influenza drugs include: ion channel M2 inhibitors, such as amantadine and rimantadine; neuraminidase inhibitors, such as oseltamivir, zanamivir, and peramivir; and other drugs, such as moroxydine and ribavirin. However, the influenza virus is prone to mutation and has developed resistance to currently common anti-influenza drugs, and thus, researching new anti-influenza drugs is a long-term need.

Deuterium (D), also known as heavy hydrogen, is a non-radioactive isotope of hydrogen. Modifying drugs with deuterium can improve the absorption, distribution, metabolism and/or excretion (ADME) properties of these drugs while retaining the biological activity of the original drugs. Therefore, researching deuterated anti-influenza drugs is a pathway to expand the options for anti-influenza medications.

### SUMMARY

The present application provides a compound and a preparation method therefor, a pharmaceutical composition of the compound, and use of the compound and the pharmaceutical composition of the compound in the treatment of influenza.

In one aspect, the present application provides a compound which is a compound of Formula X or a pharmaceutically acceptable salt thereof, 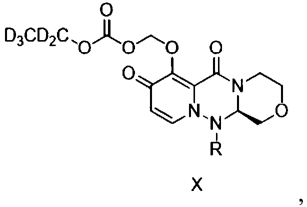 wherein R is selected from H, Fmoc or

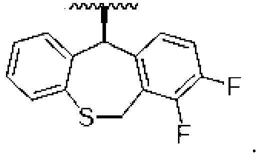

In another aspect, the present application provides a pharmaceutical composition. The pharmaceutical composition includes an effective amount of a compound of Formula X or a pharmaceutically acceptable salt thereof and a pharmaceutically acceptable adjuvant, wherein R is

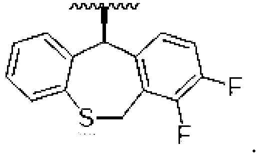

The pharmaceutically acceptable adjuvant includes a solvent, a diluent, other liquid excipients, a dispersing agent or a suspending agent, a surfactant, an isotonic agent, a thickener, an emulsifier, a preservative, a solid adhesive or a lubricant, and the like.

In some embodiments, the pharmaceutical composition includes one or more other therapeutic agents.

In some embodiments, the other therapeutic agents are selected from anti-influenza viral agents or vaccines.

In some embodiments, the other therapeutic agents are optionally selected from at least one of oseltamivir, zanamivir, peramivir, laninamivir, favipiravir, Abidol hydrochloride, amantadine, rimantadine, ribavirin, stachyflin, ingavirin, baloxavir or a vaccine.

In some embodiments, the pharmaceutical composition may be in a dosage form of a liquid, a solid, a semi-solid, a gel or a spray.

The compound of the present application or a pharmaceutically acceptable composition may be administered by any suitable means, and the above-described compound and the pharmaceutically acceptable composition may be administered to humans or other animals by oral, rectal, parenteral, intracisternal, intravaginal, intraperitoneal or topical administration according to the severity of a disease. The preferred administration method is oral administration.

The compound of the present application or the pharmaceutically acceptable salt thereof may be administered in a unit dosage form. The dosage form for administration may be a liquid dosage form or a solid dosage form. The liquid dosage form may be solutions, colloids, microparticle preparations or suspensions. Other dosage forms such as tablets, capsules, dropping pills, aerosols, pills, powders, emulsions, granules, suppositories, lyophilized powder injections, inclusion compounds, implants, patches, and liniments may also be adopted.

Oral tablets and capsules may contain excipients, for example, binders such as syrup, Arabic gum, sorbitol, tragacanth or polyvinylpyrrolidone; fillers such as sucrose, lactose, corn starch, calcium phosphate, sorbitol or aminoacetic acid; lubricants such as magnesium stearate, talcum, polyethylene glycol or silica; disintegrating agents such as potato starch; or acceptable humectants such as sodium lauryl sulfate. Tablets may be coated by using methods well known in pharmaceutics.

Oral liquids may be prepared into suspensions of water and oil, solutions, emulsions, syrups or elixirs, or may be prepared into dried products, which are supplemented with water or other suitable media before use. The liquid liquids may include conventional additives, for example, suspending agents such as sorbitol, cellulose diethyl ether or glucose syrup; emulsifiers such as lecithin, sorbitan monooleate or Arabic gum; non-aqueous adjuvants (possibly including edible oil) such as almond oil, or grease such as glycerin, ethylene glycol or ethanol; or preservatives such as methyl *p*-hydroxybenzoate, propyl *p*-hydroxybenzoate or sorbic acid. Flavoring agents or colorants may be added if desired.

The suppositories may include conventional suppository bases such as cocoa butter or other glycerides.

For parenteral administration, the liquid dosage forms are typically prepared from the compound and an adjuvant for disinfection. The preferred adjuvant is water. Depending on the chosen adjuvant and THE drug concentration, the compound may be dissolved in the adjuvant or prepared into a suspension solution. When the compound is prepared into an injectable solution, the compound is first dissolved in water, then filtered and sterilized before being sealed in vials or ampoules.

For topical application to the skin, the compound of the present application may be prepared into appropriate ointments, lotions or creams, and the active ingredient is suspended or dissolved in one or more adjuvants. The adjuvants suitable for use in ointments include, but are not limited to, mineral oil, liquid petrolatum, white petrolatum, propylene glycol, polyethylene oxide, polypropylene oxide, emulsifying wax and water. The adjuvants suitable for use in lotions and creams include, but are not limited to, mineral oil, sorbitan monostearate (Tween 60), cetyl ester wax, cetearyl alcohol, 2-octyldodecanol, benzyl alcohol and water.

In another aspect, the present application provides use of a compound of Formula X or a pharmaceutical composition of the compound of Formula X in the preparation of a drug for preventing, handling, treating or alleviating an influenza viral infection disease in a patient, wherein R is

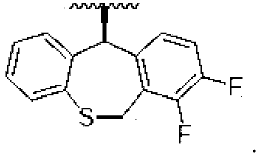

In some embodiments, the influenza viral infection is an influenza A viral infection or an influenza B viral infection.

In another aspect, the present application provides a drug prepared from a compound of Formula X or a pharmaceutical composition of the compound of Formula X for preventing, handling, treating or alleviating an influenza viral infection disease in a patient, wherein R is

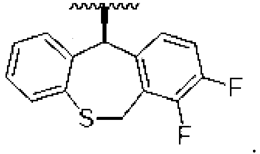

In some embodiments, the influenza viral infection is an influenza A viral infection or an influenza B viral infection.

In another aspect, the present application provides a method for using a drug for preventing, handling, treating or alleviating an influenza viral infection disease in a patient. The method includes: administering a pharmaceutically acceptable effective amount of the compound or the pharmaceutical composition of the present application to a patient.

Preferably, the influenza viral infection is an influenza A viral infection or an influenza B viral infection.

The present application further provides a method for preventing, treating or alleviating an infection caused by an influenza virus. The method includes: administering a therapeutically effective amount of the above-described compound or the pharmaceutically acceptable composition thereof to a patient in need of treatment. The pharmaceutical composition may be administrated with one or more other therapeutic agents.

In another aspect, the present application provides use of a compound of Formula X and a pharmaceutical composition of the compound of Formula X in the preparation of a drug for inhibiting RNA polymerase of an influenza virus, wherein R is

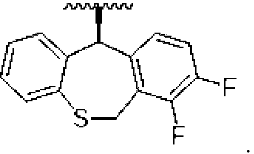

Preferably, the RNA polymerase is a cap-dependent nucleic acid PA endonuclease.

Unless otherwise specified, the present application includes stereoisomers, tautomers, nitrogen oxides, solvates, metabolites and pharmaceutically acceptable salts of the compound of the present application. The term "pharmaceutically acceptable" means that a substance or composition must be chemically and/or toxicologically compatible with other components contained in the preparation and/or the mammal to be treated.

In another aspect, the present application provides an intermediate for the preparation of a compound of Formula X. The intermediate has a structure of a compound of Formula III:

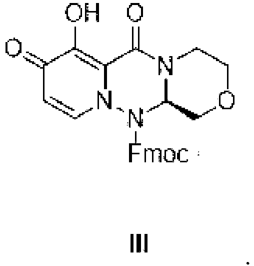

In some embodiments, the present application further provides a method for preparing a compound of Formula III. The method includes: carrying out a condensation reaction on Compound II, 9-fluorenylmethyl chloroformate, an alkali and a reaction solvent at a certain temperature, and carrying out a hydrolysis reaction in the presence of a catalyst and an organic solvent to produce a compound of Formula III:

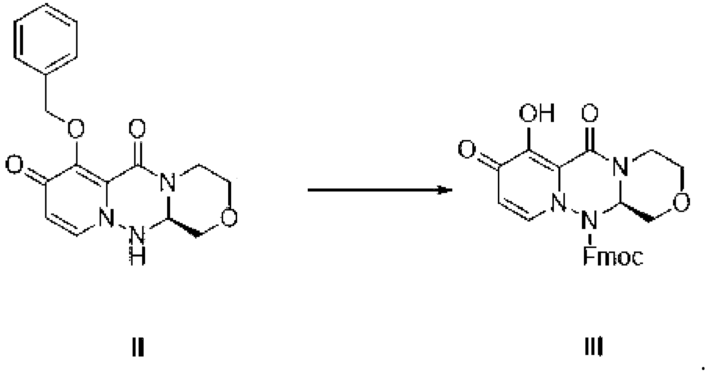

In some embodiments, the reaction solvent for the condensation reaction is selected from at least one of 1,4-dioxane, tetrahydrofuran or acetonitrile. In some embodiments, the reaction solvent is 1,4-dioxane, which facilitates the progress of the reaction and post-treatment.

In some embodiments, the condensation reaction is performed at a temperature of 0-40 °C. In some embodiments, the condensation reaction is performed at a temperature of 25 °C, which facilitates the progress of the reaction.

In some embodiments, the alkali for the condensation reaction is selected from a sodium bicarbonate solution and/or a sodium carbonate solution. In some embodiments, the alkali is a sodium bicarbonate solution, which facilitates the progress of the reaction and post-treatment.

In some embodiments, the molar ratio of Compound II to 9-fluorenylmethyl chloroformate is 1:(1-3). In some embodiments, the molar ratio of Compound II to 9-fluorenylmethyl chloroformate is 1:1, which facilitates the production and obtaining of the resulting product.

In some embodiments, the molar ratio of Compound II to the alkali is 1:(1-3). In some embodiments, the molar ratio of Compound II to the alkali is 2:3, which facilitates the production and obtaining of the resulting product.

In some embodiments, the hydrolysis reaction is performed at a temperature of 60-100 °C. In some embodiments, the hydrolysis reaction is performed at a temperature of 80 °C, which facilitates the progress of the reaction.

In some embodiments, the reaction solvent for the hydrolysis reaction is selected from N-methylpyrrolidone and/or N,N-dimethylformamide. In some embodiments, the reaction solvent is N-methylpyrrolidone, which facilitates the progress of the reaction and post-treatment.

In some embodiments, the catalyst for the hydrolysis reaction is lithium chloride, which facilitates the production and obtaining of the resulting product.

In some embodiments, a molar ratio of Compound II to the catalyst is 1:(6-12). In some embodiments, the molar ratio of Compound II to the catalyst is 1:9, which facilitates the production and obtaining of the resulting product.

After the completion of the reaction, post-treatment is optionally carried out. In some embodiments, the post-treatment includes: after the completion of the reaction, cooling the resulting mixed solution, adding water for crystallization, and filtering to obtain Compound III as an off-white solid.

In another aspect, the present application provides a method for preparing a compound of Formula X, wherein R is Fmoc. When R is Fmoc, the structure of the compound of Formula X is as shown in a compound of Formula VII: 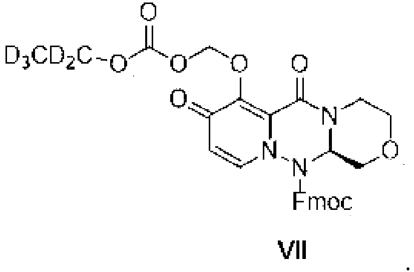 The method includes the following steps:
step one: carrying out an esterification reaction on Compound IV, Compound V, an acid-binding agent and a reaction solvent at a certain temperature to produce a compound of Formula VI; and
step two: carrying out a substitution reaction is carried out on Compound III, Compound VI, an alkali, a catalyst and a reaction solvent at a certain temperature to produce a compound of Formula VII;

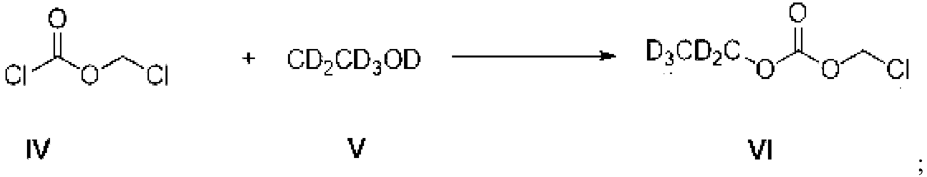

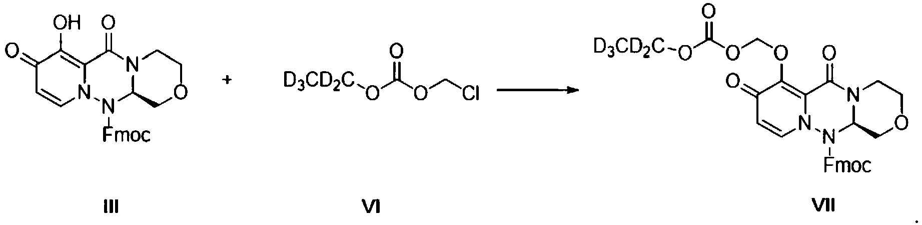

In step one, the reaction solvent is selected from at least one of dichloromethane, anhydrous diethyl ether or tetrahydrofuran. In some embodiments, the reaction solvent is dichloromethane, which facilitates the progress of the reaction and post-treatment.

In step one, the reaction is performed at a temperature of -10-40 °C. In some embodiments, the reaction is performed at a temperature of 0-30 °C, which facilitates the progress of the reaction.

In step one, the acid-binding agent is selected from pyridine and/or triethylamine. In some embodiments, the acid-binding agent is pyridine, which facilitates the production and obtaining of the resulting product.

In step one, a molar ratio of Compound IV to Compound V is 1:(0.8-1.5). In some embodiments, the molar ratio of Compound IV to Compound V is 1:1, which facilitates the production and obtaining of the resulting product.

In step one, a molar ratio of Compound IV to the acid-binding agent to the acid is 1:(1-2). In some embodiments, the molar ratio of Compound IV to the acid-binding agent is 1:1.2, which facilitates the production and obtaining of the resulting product.

In step one, after the completion of the reaction, the post-treatment is optionally carried out. In some embodiments, the post-treatment includes: after the completion of the reaction, quenching the reaction solution, allowing the quenched reaction solution to stand for liquid separation, collecting an organic phase, washing the organic phase with saturated brine, drying the organic phase with anhydrous sodium sulfate, and concentrating to obtain Compound VI.

In step two, the reaction solvent is selected from at least one of *N*,*N*-dimethylformamide, *N*,*N*-dimethylacetamide or acetone. In some embodiments, the reaction solvent is *N*,*N*-dimethylformamide, which facilitates the progress of the reaction and post-treatment.

In step two, the reaction is performed at a temperature of 40-70 °C. In some embodiments, the reaction is performed at a temperature of 60 °C, which facilitates the progress of the reaction.

In step two, the alkali is selected from at least one of potassium carbonate, cesium carbonate, potassium phosphate or sodium carbonate. In some embodiments, the alkali is potassium carbonate, which facilitates the progress of the reaction and post-treatment.

In step two, the catalyst is selected from at least one of potassium iodide, sodium iodide or potassium bromide. In some embodiments, the catalyst is potassium iodide, which facilitates the progress of the reaction and post-treatment.

In step two, a molar ratio of Compound III to Compound VI is 1:(1-3). In some embodiments, the molar ratio of Compound III to Compound VI is 1:2, which facilitates the production and obtaining of the resulting product.

In step two, a molar ratio of Compound III to the alkali is 1:(1-3). In some embodiments, the molar ratio of Compound III to the alkali is 1:2, which facilitates the production and obtaining of the resulting product.

In step two, a molar ratio of Compound III to the catalyst is 1:(0.1-3). In some embodiments, the molar ratio of Compound III to the catalyst is 1:0.5, which facilitates the production and obtaining of the resulting product.

In step two, after the completion of the reaction, the post-treatment is optionally carried out. In some embodiments, the post-treatment includes: after the completion of the reaction, cooling the resulting mixed solution, adding water for crystallization, filtering, and drying to obtain Compound VII as a light yellow solid.

In another aspect, the present application provides a method for preparing a compound of Formula X, wherein R is H. When R is H, the structure of the compound of Formula X is as shown in a compound of Formula VIII: 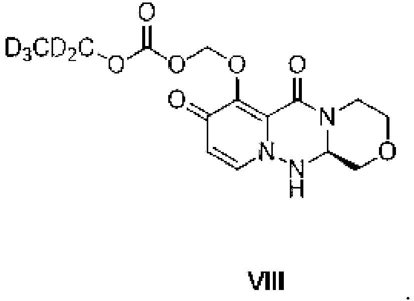 The method includes: carrying out a deprotection reaction on Compound VII, an alkali and an organic solvent at a certain temperature to produce a compound of Formula VIII:

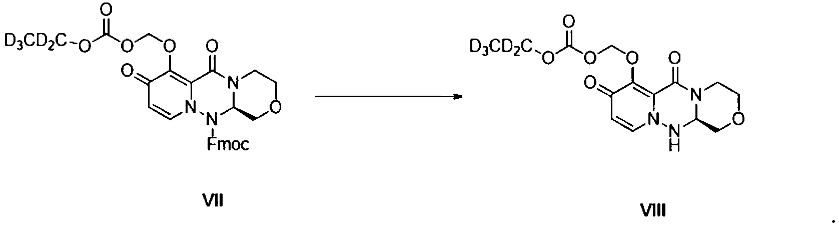

In some embodiments, a solvent of the reaction is dichloromethane, which facilitates the progress of the reaction and post-treatment.

In some embodiments, the reaction is performed at a temperature of 10-40 °C. In some embodiments, the reaction is performed at a temperature of 20 °C, which facilitates the progress of the reaction.

In some embodiments, the alkali is selected from at least one of hexahydropyridine, concentrated ammonia water, piperidine or 1,8-diazabicyclo[5.4.0]undec-7-ene. In some embodiments, the alkali is hexahydropyridine, which facilitates the production and obtaining of the resulting product.

In some embodiments, a molar ratio of Compound VII to the alkali is 1:(0.5-3). In some embodiments, the molar ratio of Compound VII to the alkali is 1:1, which facilitates the production and obtaining of the resulting product.

After the completion of the reaction, the post-treatment is optionally carried out. In some embodiments, the post-treatment includes: adding a solvent to the resulting solid mixture, recrystallizing, filtering, and drying to obtain Compound VIII as a white solid.

In another aspect, the present application provides a method for preparing a compound of Formula X, wherein R is 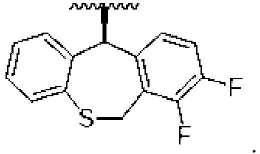 When R is 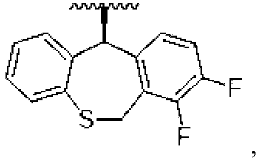 the structure of the compound of Formula X is as shown in a compound of Formula I. The preparation method includes: carrying out a condensation reaction on Compound VIII, Compound IX, a condensing agent, a catalyst and an organic solvent at a certain temperature to produce a compound of Formula I:

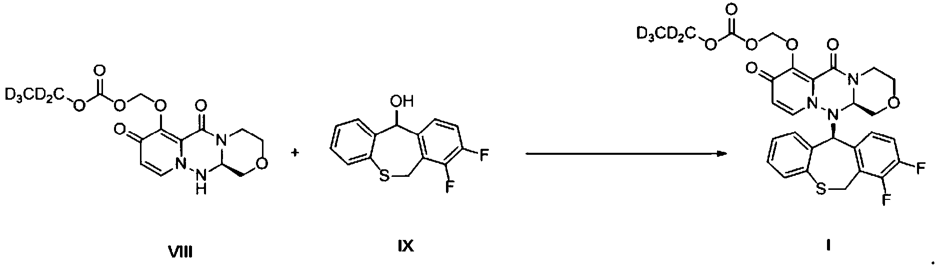

In some embodiments, a solvent of the reaction is ethyl acetate and/or cyclohexane, which facilitates the progress of the reaction and post-treatment.

In some embodiments, the reaction is performed at a temperature of 60-80 °C. In some embodiments, the reaction is performed at a temperature of 70 °C, which facilitates the progress of the reaction.

In some embodiments, the condensing agent is selected from at least one of 1-ethyl-3-(3-dimethylaminopropyl) carbodiimide hydrochloride, I-propylphosphonic anhydride or O-(7-azabenzotriazol-1-yl)-*N*,*N*,*N*',*N*'-tetramethyluronium hexafluorophosphate. In some embodiments, the catalyst is 1-propylphosphonic anhydride, which facilitates the production and obtaining of the resulting product.

In some embodiments, the catalyst is methanesulfonic acid, which facilitates the production and obtaining of the resulting product.

In some embodiments, a molar ratio of Compound VIII to Compound IX is 1:(1-3). In some embodiments, the molar ratio of Compound VIII to Compound IX is 1:1.2, which facilitates the production and obtaining of the resulting product.

In some embodiments, a molar ratio of Compound VIII to the condensing agent is 1:(1-4). In some embodiments, the molar ratio of Compound VIII to the condensing agent is 1:1.5, which facilitates the production and obtaining of the resulting product.

In some embodiments, a molar ratio of Compound VIII to the catalyst is 1:(0.5-4). In some embodiments, the molar ratio of Compound VIII to the catalyst is 1:2.5, which facilitates the production and obtaining of the resulting product.

After the completion of the reaction, the post-treatment is optionally carried out. In some embodiments, the post-treatment includes: after the completion of the reaction, cooling the resulting mixed solution, extracting, drying, recrystallizing, filtering, and drying to obtain Compound I as an off-white solid.

In another aspect, the present application provides a method for preparing a compound of Formula X, wherein R is 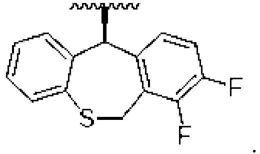 The method includes the following steps:
step one: carrying out a condensation reaction on Compound II, 9-fluorenylmethyl chloroformate, sodium bicarbonate and 1,4-dioxane at 0-40 °C, and a hydrolysis reaction is carried out with lithium chloride and *N*-methylpyrrolidone at 60-100 °C to produce a compound of Formula III;
step two: carrying out an esterification reaction on Compound IV, Compound V, pyridine and dichloromethane at -10-40 °C to produce a compound of Formula VI; and carrying out a substitution reaction on Compound III, Compound VI, potassium carbonate, potassium iodide and *N*,*N*-dimethylformamide at 40-70 °C to produce a compound of Formula VII;
step three: carrying out a deprotection reaction on Compound VII, hexahydropyridine and dichloromethane at 10-40 °C to produce a compound of Formula VIII;
step four: carrying out a condensation reaction on Compound VIII, Compound IX, 1-propyl phosphoric acid cyclic anhydride, methanesulfonic acid, ethyl acetate and cyclohexane at 60-80 °C to produce a compound of Formula I;

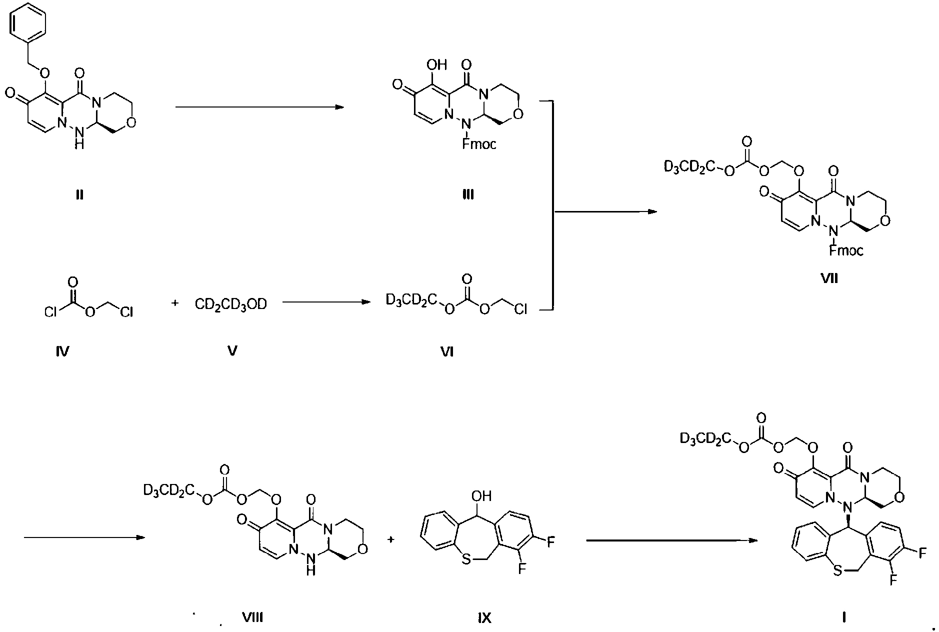

It is to be understood that in case of any discrepancy between the chemical names and chemical structures provided in the specification, the structures shall prevail.

Compared to existing compounds of the same class, Compound I of the present application exhibits superior pharmacological activity. Therefore, Compound I provided herein demonstrates more favorable druggability relative to the existing compounds of the same class.

In summary, the present application provides a compound and a preparation method therefor. Compound I specifically relates to a cap-dependent nucleic acid PA endonuclease inhibitor, and Compound III, Compound VII and Compound VIII may be used as intermediates for the preparation of Compound I. The present application further provides a pharmaceutical composition of Compound I and the use of Compound I and the pharmaceutical composition of Compound I in the treatment of influenza. The compound is suitable for being prepared into drugs of various dosage forms and can be widely used for treating seasonal influenza.

### BRIEF DESCRIPTION OF DRAWINGS

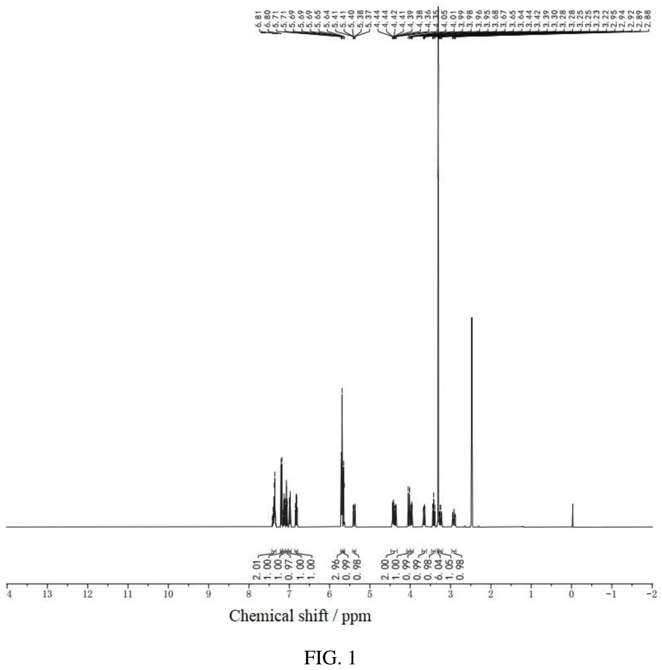

FIG. 1 is a proton nuclear magnetic resonance spectrum of Compound I prepared in Example 5 of the present application.

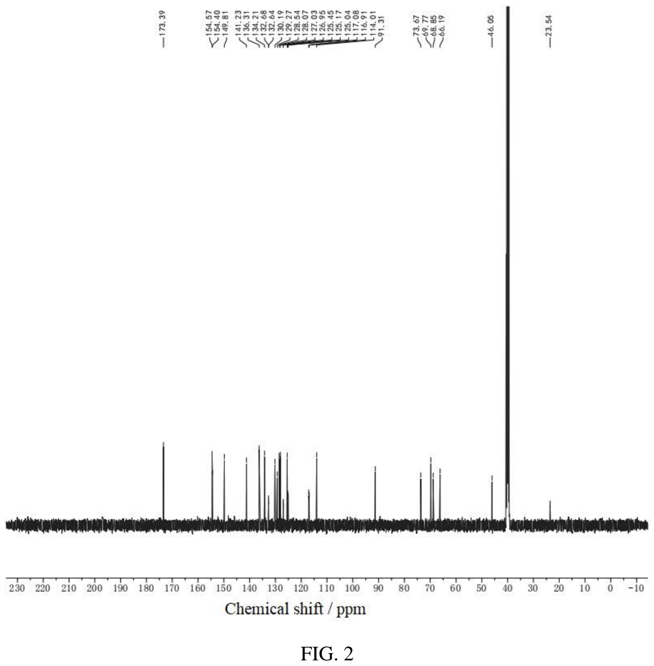

FIG. 2 is a carbon-13 nuclear magnetic resonance spectrum of Compound I prepared in Example 5 of the present application.

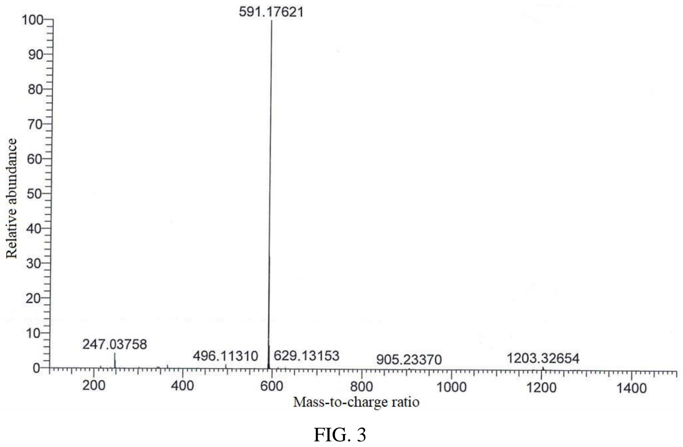

FIG. 3 is a mass spectrogram of Compound I prepared in Example 5 of the present application.

### DETAILED DESCRIPTION

### Definitions and General Terms

The term "include" or "comprise" used herein is an open-ended expression, meaning it includes the content specified in the present application but does not exclude other aspects.

The term "stereoisomer" includes enantiomers, diastereoisomers, conformational isomers (rotamers), geometric isomers (cis/trans isomers), atropisomers, and the like. Depending on the choice of starting materials and methods, the compound of the present application may exist in the form of one of the possible isomers or mixtures thereof, such as racemates and mixtures of diastereoisomers (which depend on the number of asymmetric carbon atoms).

The term "pharmaceutically acceptable salt" refers to organic and inorganic salts of the compound of the present application.

The compound of the present application, including salts thereof, may also be obtained in the form of hydrates thereof or may include other solvents used in its crystallization. The compound of the present application may inherently or by design form solvates with pharmaceutically acceptable solvents (including water); therefore, the present application also includes both solvated and unsolvated forms of the compound.

The term "effective amount" refers to an amount of the compound of the present application that elicits the intended biological response. In the present application, the intended biological responses include reducing the amount of influenza viruses, alleviating or ameliorating the severity, duration, progression or onset of an influenza virus infection, preventing the spread of an influenza virus infection, preventing the recurrence, evolution, onset or progression of symptoms associated with an influenza virus infection, or enhancing or improving the preventive or therapeutic effects of another anti-influenza infection therapy used.

The terms "first" and "second" are only for the purpose of illustration, rather than being interpreted as indicating or implying any relative importance, or implicitly indicating the number of indicated technical features. Therefore, the features defined with "first" and "second" may explicitly or implicitly include one or more of such features. As described herein, "plurality" or "multiple" refers to two or more, unless otherwise expressly specified and limited.

The description with reference to terms "one embodiment", "some embodiments", "example", "specific example" or "some examples" means that specific features, structures, materials or characteristics described in connection with the embodiment or example are included in at least one embodiment or example of the present application. In the specification, the illustrative description of the preceding terms does not necessarily refer to the same embodiment or example. Furthermore, the described specific features, structures, materials or characteristics may be involved in any one or more embodiments in a proper way. Moreover, the different embodiments or examples described in the specification and the features of the different embodiments or examples may be incorporated and combined by those skilled in the art if there is no contradiction.

In the present application, the expressions "Compound I", "compound represented by Formula I" and "Formula I" represent the same compound.

In the present application, "optional" or "optionally" indicates that something may be included or excluded, or an action may be performed or not performed. For example, "optionally adding a reaction solvent to the crude product obtained in step one" means that the reaction solvent may or may not be added to the crude product obtained in step one.

To enable those skilled in the art to better understand the technical solutions of the present application, some non-limiting examples are further disclosed below to provide a more detailed description of the present application.

All reagents used in the present application may be purchased commercially or prepared by the methods described herein.

In the present application, a reaction is considered complete when the remaining amount of the starting material does not exceed 5%, 3%, 2%, 1%, or 0.5% of the initial charge.

In the present application, the meaning of the statistical P-value ranges is as follows: 0.01 < P < 0.05 indicates a significant difference, typically marked with "×"; and P < 0.01 indicates a highly significant difference, typically marked with "××".

The T-3 compound and T-4 compound used in the present application are prepared according to the methods described in Patent CN110317211A.

### Preparation Example

### Example 1 Preparation of (9H-fluoren-9-yl)methyl (R)-7-hydroxy-6,8-dioxo-1,3,4,6,8,12a-hexahydro-12H-[1,4]oxazino[3,4-c]pyrido[2,1-f][1,2,4]t riazine-12-carboxylate (Compound III)

(R)-7-(benzyloxy)-3,4,12,12a-tetrahydro-1H-[1,4]oxazino[3,4-c]pyrido[2,1-f][1,2,4]tri azine-6,8-dione (Compound II, 6.5 g, 20 mmol), 9-fluorenylmethyl chloroformate (5.6 g, 21 mmol), 1,4-dioxane (65.1 g) and a 10% sodium bicarbonate solution (25.2 g, 30 mmol) were added to a reaction flask. The reaction system reacted at 25 °C for 4 h. Dichloromethane (195.7 g) was added to the reaction system, and the resulting reaction system was extracted three times. The organic phases were combined, purified water (125.8 g) was added, and the resulting mixture was stirred, washed twice, and allowed to stand for liquid separation. The resulting organic phase was collected, anhydrous sodium sulfate (22.6 g) was added, and the resulting mixture was stirred for drying for 1 h and filtered. The filtrate was collected and spun to dryness at 50 °C. *N*-methylpyrrolidone (76.9 g) and lithium chloride (7.6 g, 180 mmol) were added to the residue, and the resulting mixture was stirred and heated to 80 °C to react for 12 h. The temperature was then lowered to 10 °C, and purified water (210.7 g) was added dropwise to the reaction system. The resulting reaction system was cooled to 0-20 °C, crystallized for 1 h and filtered to obtain 7.4 g of Compound III (off-white solid, purity: 95%, yield: 81.0%).

¹HNMR (CDCl₃) δ: 10.10 (1H, s), 7.79 (2H, d), 7.62 (2H, m), 7.34-7.43 (4H, m), 6.96 (1H, d), 6.28 (1H, d), 5.57 (1H, m), 4.70 (2H, d), 4.46 (1H, d), 4.02 (1H, m), 3.77 (1H, m), 3.40-3.64 (4H, m).

### Example 2 Preparation of chloromethyl (ethyl-d5) carbonate (Compound VI)

Compound IV (5.0 g, 38.8 mmol) and pyridine (3.4 g, 43 mmol) were added to dichloromethane (125.7 g), and the resulting mixed solution was cooled to -5 °C. Compound V (2.4 g, 46.1 mmol) dissolved in dichloromethane (10.1 g) was added dropwise to the above mixed solution. After the addition was complete, the reaction temperature was raised to 0 °C, the reaction was carried out for 0.5 h with the reaction temperature maintained, then the reaction temperature was further raised to 20 °C, and the reaction was carried out for 2 h. Upon reaction completion, the reaction was quenched with dilute hydrochloric acid (2 mol/L, 40 mL), and the resulting mixture was allowed to stand for liquid separation. The organic phase was collected, washed with saturated brine (40 mL × 2), dried with anhydrous sodium sulfate (22.6 g), filtered and concentrated to obtain 5.0 g of Compound VI (pale yellow liquid, purity: 97%, yield: 90%).

### Example 3 Preparation of (9H-fluoren-9-yl)methyl (R)-7-(((ethoxy-d5)carbonyl)oxy)methoxy)-6,8-dioxo-1,3,4,6,8,12a-hexahydro-12H-[1,4]oxazi no[3,4-c]pyrido[2,1-f][1,2,4]triazine-12-carboxylate (Compound VII)

Compound III (7.2 g, 15.7 mmol), Compound VI (4.5 g, 31.3 mmol), potassium carbonate (4.4 g, 31.8 mmol) and potassium iodide (1.3 g, 7.8 mmol) were successively added to N,N-dimethylformamide (72.0 g) and mixed. The resulting mixed solution was heated to 60 °C and reacted for 12 h. After the mixed solution was cooled to 0 °C, water (150 mL) was added for crystallization. The resulting mixture was filtered, and the resulting solid was dried to obtain 7.5 g of Compound VII as a solid (pale yellow solid, purity: 96%, yield: 87%).

¹HNMR (CDCl₃) δ: 7.90 (2H, d), 7.65 (2H, m), 7.28-7.38 (4H, m), 6.99 (1H, d), 6.83 (2H, m), 6.33 (1H, d), 5.61 (1H, m), 4.75 (2H, d), 4.50 (1H, d), 4.05 (1H, m), 3.81 (1H, m), 3.45-3.66 (4H, m).

### Example 4 Preparation of (R)-(((6,8-dioxo-3,4,6,8,12,12a-hexahydro-1H-[1,4]oxazino[3,4-c]pyrido[2,1-f][1,2,4]triazin-7-yl)oxy)methyl (ethyl-d5) carbonate (Compound VIII)

Compound VII (7.0 g, 12.7 mmol), hexahydropyridine (1.1 g, 12.9 mmol) and dichloromethane (186.2 g) were successively added to a reaction flask. The reaction system was stirred at 20 °C for 2 h and spun to dryness at 50 °C. Methanol (126.1 g) was added to the reaction system for recrystallization, and the resulting mixture was filtered and dried under vacuum at 60 °C to obtain 3.9 g of Compound VIII (off-white solid, purity: 98%, yield: 90%).

¹HNMR (CDCl₃) δ: 6.97 (1H, d), 6.79 (2H, m), 6.41 (1H, m), 6.31 (1H, d), 5.57 (1H, m), 3.98 (1H, m), 3.73 (1H, m), 3.40-3.57 (4H, m).

### Example 5 Preparation of [[(12aR)-12-[(11S)-7,8-difluoro-6,11-dihydrodibenzo[b,e]thiepin-11-yl]-6,8-dioxo-3,4,6,8,12,12 a-hexahydro-1H-[1,4]oxazino[3,4-c]pyrido[2,1-f][1,2,4]triazin-7-yl]oxy]methyl (ethyl-d5) carbonate (Compound I)

Ethyl acetate (12.8 g), cyclohexane (4.5 g), Compound VIII (3.0 g, 8.7 mmol) and Compound IX (2.8 g, 10.6 mmol) were successively added to a reaction flask. 1-propyl phosphoric acid cyclic anhydride (50% solution in ethyl acetate) (8.3 g, 13.0 mol) was added dropwise to the reaction system under stirring, and then methanesulfonic acid (2.1 g, 21.8 mmol) was added dropwise to the reaction system. The reaction system was heated to 70 °C and reacted for 24 h. After the reaction system was cooled to 20 °C, a 20% sodium hydroxide solution was added dropwise to the reaction system under stirring to adjust the pH of the reaction system to 7. The resulting mixture was allowed to stand for liquid separation. The upper organic phase was collected. The aqueous phase was extracted with ethyl acetate (30.0 g) under stirring and allowed to stand for liquid separation to collect an upper organic phase. The organic phases were combined, dried with anhydrous sodium sulfate (22.6 g) for 1 h and filtered. The filtrate was spun to dryness at 60 °C to obtain a brown oil. Dichloromethane (24.1 g) was added to dissolve the brown oil, and n-heptane (26.5 g) was added dropwise to the reaction solution under stirring. The resulting mixture was cooled to 10 °C, stirred for crystallization for 1 h and filtered. The filter cake was dried under vacuum at 60 °C to obtain 4.0 g of Compound I as a solid (off-white solid, purity: 98%, yield: 78%).

¹HNMR (DMSO-d6) δ: 7.34-7.42 (2H, m), 7.19 (1H, dd), 7.15 (1H, m), 7.13 (1H, m), 7.07-7.11 (1H, dd), 5.51-5.71 (3H, m), 5.37-5.41 (1H, dd), 4.35-4.44 (2H, m), 3.95-4.05 (2H, ddd), 3.64-3.68 (1H, dd), 3.44-3.54 (2H, m), 3.42 (1H, t), 3.22-3.28 (1H, m), 2.92 (1H, m).

### Activity Test Example

### Example A In vitro anti-influenza virus pharmacodynamics experiment

This example studied the inhibitory effects of the compound of Formula I against influenza A virus (H3N2) and influenza B virus (IBV) *in vitro.*

The *in vitro* cytotoxic effects of the test compound (the compound of Formula I) and the positive control drugs (baloxavir marboxil, baloxavir, T-3 compound and T-4 compound) on Madin-Darby canine kidney (MDCK) cells were determined using a Cell Counting Kit (CCK-8). MDCK cells were seeded in 96-well plates and cultured overnight. After different concentrations of the drugs were added, the cells were incubated for 48 h. CCK-8 was then added, and the cells were incubated in the dark for 14 h. The optical density (OD) at 450 nm (OD₄₅₀ₙₘ) values were measured using a microplate reader, and the half-maximal inhibitory concentration (IC₅₀) of the drugs on the cells was calculated.

The *in vitro* inhibitory effects of the test compound (the compound of Formula I) and the positive control drugs (baloxavir marboxil, baloxavir, T-3 compound and T-4 compound) against the influenza A virus subtype H3N2 strain (A/California/2/2014) and the influenza B virus strain (B/Florida/78/2015) were determined using the cytopathic effect (CPE) assay. MDCK cells were seeded in 96-well plates and cultured overnight. The cells were infected with 100 TCID₅₀ (Influenza A: 10^{-0.13}/0.1mL; Influenza B: 10^{-0.29}/0.1mL) of virus by adsorption for 2 h. Then, media containing different concentrations of the drugs were added to continue the culture, and incubation was terminated when the cytopathic effects in the virus control groups reached approximately 75%. CCK-8 was then added, and the cells were incubated in the dark for 1-4 h. The OD₄₅₀ₙₘ values were measured, and the half-maximal effective concentration (EC₅₀) of the drugs against the viruses was calculated.

### Experiment results:

*In vitro* cytotoxicity result: The compound of Formula I, baloxavir marboxil, baloxavir, T-3 compound and T-4 compound all exhibited certain cytotoxic effects on MDCK cells, with IC₅₀ values of 17.36 µM, 11.61 µM, 10.45 µM, 9.21 µM and 12.30 µM, respectively. These results indicate that the compound of Formula I demonstrated the most favorable safety profile toward normal cells.

*In vitro* antiviral activity result: At a concentration of 250 nM, the compound of Formula I can provide significant protection against influenza virus-induced cell damage and inhibit viral cytopathic effects. The EC₅₀ values of the compound of Formula I against the H3N2 virus and the IBV virus were 5.02 nM and 8.15 nM, respectively.

The EC₅₀ values of the compound of Formula I, baloxavir marboxil, baloxavir, T-3 compound and T-4 compound against the influenza A virus subtype H3N2 strain (A/California/2/2014) were 5.02 nM, 13.42 nM, 1.31 nM, 8.54 nM and 7.21 nM, respectively, suggesting the following order of anti-influenza A virus potency: baloxavir marboxil < T-3 compound < T-4 compound < Compound of Formula I < baloxavir. The EC₅₀ values of the compound of Formula I, baloxavir marboxil, baloxavir, T-3 compound and T-4 compound against the influenza B virus strain (B/Florida/78/2015) were 8.15 nM, 15.39 nM, 2.06 nM, 10.41 nM, and 11.98 nM, respectively, suggesting the following order of anti-influenza B virus potency: baloxavir marboxil < T-4 compound < T-3 compound < Compound of Formula I < baloxavir.

### Example B In vivo anti-influenza virus pharmacodynamics study

In this example, the H3N2 influenza A virus and the IBV influenza B virus were used to intranasally infect BALB/c mice to establish an influenza A virus infection model and an influenza B virus infection model. After infection, the compound of Formula I was administered to the mice by oral gavage. Changes in lung index and lung viral load of the mice were observed. Oseltamivir phosphate granules, baloxavir marboxil, T-3 compound and T-4 compound were set as positive controls to evaluate the *in vivo* efficacy of the compound of Formula I against influenza viruses.

### (1) In vivo pharmacodynamics study of the compound of Formula I against the H3N2 influenza A virus

A total of 250 qualified SPF-grade BALB/c mice, weighing 11.9-15.1 g, half male and half female, were randomly divided into 7 groups based on genders and body weights: normal control group, model control group, oseltamivir phosphate granules group (6 mg/kg), baloxavir marboxil group (6 mg/kg), T-3 compound group (6 mg/kg), T-4 compound group (6 mg/kg) and Compound of Formula I group (6 mg/kg). The normal control group, oseltamivir phosphate granules group, baloxavir marboxil group, T-3 compound group (6 mg/kg) and T-4 compound group each included 30 mice, and the model control group and the Compound of Formula I group each included 50 mice. Two hours before the first administration, all mice were lightly anesthetized with ether. Except for the normal control group, all mice were intranasally inoculated with the stock solution of H3N2 influenza A virus (viral titer: 6 log₂) at 100 µL per mouse. The normal control group was administrated with an equal volume of phosphate-buffered solution intranasally. Each group of mice was administered the corresponding concentration of the drug solution at 20 mL/kg, once daily for 5 consecutive days. The normal control group and the model control group were administered an equal volume of 0.5% methylcellulose (0.5% MC) by oral gavage. The body weights of the mice in each group were recorded daily. At 24 h and 48 h after administration, 10 mice (half male and half female) were randomly selected from each group and euthanized by cervical dislocation, and their lungs were dissected and weighed to calculate the lung index. The influenza A viral load in the lung tissue of the mice was detected using a real-time quantitative polymerase chain reaction (PCR) method. In the model control group and the Compound of Formula I group, 10 mice were euthanized at 12 h, 24 h, 48 h, 72 h, and 5 days after administration. The left lung was collected to assess the H3N2 influenza A viral load. The results are shown in Table 1 and Table 2, among which Table 1 shows the effect of the compound of Formula I on the lung index of mice infected with influenza A virus (*x̅* ± *s*, n = 10), and Table 2 shows the effect of the compound of Formula I on the viral load in the lung tissue of mice infected with H3N2 influenza A virus (*x̅* ± *s*, n = 10).

**Table 1**

| Group | Dose (mg/kg) | Lung index (mg/g) | |
|---|---|---|---|
| | | 24 h after administration | 48 h after administration |
| Normal control group | -- | 8.17 ± 0.82 | 8.30 ± 0.74 |
| Model control group | -- | 9.93 ± 1.00⁺⁺ | 9.84 ± 1.20⁺⁺ |
| Oseltamivir phosphate granules group | 6 | 9.60 ± 0.84 | 9.18 ± 0.79 |
| Baloxavir marboxil group | 6 | 9.53 ± 0.80 | 9.11 ± 0.96 |
| T-3 compound group | 6 | 9.22 ± 1.75 | 9.31 ± 0.88 |
| T-4 compound group | 6 | 9.05 ± 0.88^{×} | 9.19 ± 0.68 |
| Compound of Formula I group | 6 | 8.67 ± 0.51^{××} | 9.00 ± 0.57^{×} |

| | | | |
|---|---|---|---|
| Note: Compared to the normal control group, ⁺⁺*P* ≤ 0.01; compared to the model control group, ^{×}*P ≤* 0.05, and ^{××}*P* ≤ 0.01. | | | |

**Table 2**

| Group | Dose (mg/kg) | PCR viral load (copies) | |
|---|---|---|---|
| | | 24 h after administration | 48 h after administration |
| Normal control group | -- | 0 ± 0 | 0 ± 0 |
| Model control group | -- | 3932981 ± 1735449⁺⁺ | 466653 ± 241352⁺⁺ |
| Oseltamivir phosphate granules group | 6 | 4117054 ± 1967261 | 146362 ± 110738^{××} |
| Baloxavir marboxil group | 6 | 1919953 ± 784647^{××} | 320599 ± 202529 |
| T-3 compound group | 6 | 1659962 ± 1018455^{××} | 229587 ± 168952^{××} |
| T-4 compound group | 6 | 1601855 ± 941760^{××} | 218389 ± 136823^{××} |
| Compound of Formula I group | 6 | 1308927 ± 1042821^{××} | 193017 ± 192655^{××} |

| | | | |
|---|---|---|---|
| Note: Compared to the normal control group, ⁺⁺*P* ≤ 0.01; compared to the model control group, ^{×}*P ≤* 0.05, and ^{××}*P* ≤ 0.01. | | | |

The above results indicate that the compound of Formula I can suppress the increase in the lung index in mice infected with the influenza A virus and significantly inhibit the replication of the influenza A virus in the lungs of the mice, suggesting that the compound of Formula I has marked *in vivo* efficacy against the influenza A virus and at an equivalent dose, the *in vivo* anti-influenza A virus efficacy of the compound of Formula I is superior to that of the control compounds.

### (2) In vivo pharmacodynamics study of the compound of Formula I against the IBV influenza B virus

A total of 210 qualified SPF-grade BALB/c mice, weighing 13.0-15.4 g, half male and half female, were randomly divided into 7 groups based on genders and body weights: normal control group, model control group, oseltamivir phosphate granules group (6 mg/kg), baloxavir marboxil group (6 mg/kg), T-3 compound group (6 mg/kg), T-4 compound group (6 mg/kg) and Compound of Formula I group (6 mg/kg), 30 mice per group. Two hours before the first administration, all mice were lightly anesthetized with ether. Except for the normal control group, all mice were intranasally inoculated with the stock solution of IBV influenza B virus (viral titer: 8 log₂) at 100 µL per mouse. The normal control group was administrated with an equal volume of phosphate-buffered solution intranasally. Each group of mice was administered the corresponding concentration of the drug solution at 20 mL/kg by oral gavage, once daily for 5 consecutive days. The normal control group and the model control group were administered an equal volume of 0.5% MC. The body weights of the mice in each group were recorded daily. At 24 h and 48 h after administration, 10 mice (half male and half female) were randomly selected from each group and euthanized by cervical dislocation, and their lungs were dissected and weighed to calculate the lung index. The influenza B viral load in the lung tissue of the mice was detected using a relative fluorescence quantitative PCR method. The results are shown in Table 3 and Table 4, among which Table 3 shows the effect of the compound of Formula I on the lung index of mice infected with IBV influenza B virus (*x̅* ± *s*, n = 10), and Table 4 shows the effect of the compound of Formula I on the viral load in the lung tissue of mice infected with IBV influenza B virus (*x̅* ± *s*, n = 10).

**Table 3**

| Group | Dose (mg/kg) | Lung index (mg/g) | |
|---|---|---|---|
| | | 24 h after administration | 48 h after administration |
| Normal control group | -- | 10.23 ± 0.88 | 9.98 ± 0.90 |
| Model control group | -- | 12.36 ± 1.01⁺⁺ | 14.64 ± 3.47⁺⁺ |
| Oseltamivir phosphate granules group | 6 | 12.52 ± 0.49 | 12.36 ± 1.53^{×} |
| Baloxavir marboxil group | 6 | 11.64 ± 1.06 | 11.45 ± 0.66^{×} |
| T-3 compound group | 6 | 12.19 ± 0.94 | 10.28 ± 0.74^{××} |
| T-4 compound group | 6 | 12.21 ± 1.58 | 10.03 ± 0.99^{××} |
| Compound of Formula I group | 6 | 12.01 ± 1.09 | 9.79 ± 0.90^{××} |

| | | | |
|---|---|---|---|
| Note: Compared to the normal control group, ⁺⁺*P* ≤ 0.01; compared to the model control group, ^{×}*P ≤* 0.05, and ^{××}*P* ≤ 0.01. | | | |

**Table 4**

| Group | Dose (mg/kg) | PCR relative quantification (Log10) | |
|---|---|---|---|
| | | 24 h after administration | 48 h after administration |
| Normal control group | -- | 1.02 ± 0.02 | 1.01 ± 0.01 |
| Model control group | -- | 14516.40 ± 12859.83⁺⁺ | 26232.89 ± 20273.22⁺⁺ |
| Oseltamivir phosphate granules group | 6 | 17092.00 ± 12844.41 | 30221.96 ± 27089.35 |
| Baloxavir marboxil group | 6 | 5586.69 ± 4192.26 | 4832.99 ± 3057.80^{××} |
| T-3 compound group | 6 | 3169.42 ± 3381.26^{×} | 2828.50 ± 1925.60^{××} |
| T-4 compound group | 6 | 2157.32 ± 2253.08^{××} | 1800.18 ± 1418.33^{××} |
| Compound of Formula I group | 18 | 1416.57 ± 1122.12^{××} | 1206.66 ± 1034.87^{××} |

| | | | |
|---|---|---|---|
| Note: Compared to the normal control group, ⁺⁺*P* ≤ 0.01; compared to the model control group, ^{×}*P* ≤ 0.05, and ^{××}*P* ≤ 0.01. | | | |

The above results indicate that the compound of Formula I can suppress the increase in the lung index in mice infected with the IBV influenza B virus and significantly inhibit the replication of the IBV influenza B virus in the lungs of the mice, suggesting that the compound of Formula I has marked *in vivo* efficacy against the influenza B virus and at an equivalent dose, the *in vivo* anti-influenza B virus efficacy of the compound of Formula I is superior to that of the control compounds.

### Example C Pharmacokinetics experiment

This example investigated the pharmacokinetics profile of the compound of Formula I administered as a methylcellulose (MC) suspension by oral gavage in rats.

The drug concentration of baloxavir was determined using a validated LC-MS/MS analytical method. Plasma samples were precipitated with acetonitrile, and the supernatant was injected for analysis. A chromatographic column, Waters XBridge C8 2.5 µm 2.1×50 mm Column, was used, and a gradient elution was carried out using a mobile phase of 0.1% formic acid in water and acetonitrile (containing 0.1% formic acid), with a flow rate of 0.60 mL/min. Monitoring was carried out using an electrospray ionization (ESI) source in a positive ion mode with a multiple reaction monitoring (MRM) method. The linear range for baloxavir was 0.1000 to 100.0 ng/mL, with a lower limit of quantification of 0.1000 ng/mL; the linear range for baloxavir marboxil was 0.1000 to 5.000 ng/mL, with a lower limit of quantification of 0.1000 ng/mL; the linear range for the compound of Formula I was 0.1000 to 5.000 ng/mL, with a lower limit of quantification of 0.1000 ng/mL; the linear range for the T-3 compound was 0.1000 to 5.000 ng/mL, with a lower limit of quantification of 0.1000 ng/mL; and the linear range for the T-4 compound was 0.1000 to 5.000 ng/mL, with a lower limit of quantification of 0.1000 ng/mL.

This experiment used the marketed drug baloxavir marboxil and the analogous compounds T-3 and T-4 as references to investigate the differences in single-dose pharmacokinetics characteristics between the compound of Formula I and the above compounds. The dose groups were designed as follows: baloxavir marboxil group, with a medium dose (0.9 mg/kg, calculated as baloxavir), T-3 and T-4 compound groups, with a medium dose (0.9 mg/kg), and Compound of Formula I group, with a medium dose (0.9 mg/kg). Blood samples (approximately 0.2 mL) were collected via the orbital plexus before administration (0 h) and at 5 min, 15 min, 30 min, 1 h, 2 h, 4 h, 6 h, 8 h, 12 h, and 24 h after administration. Plasma was separated by centrifugation at 1700 g for 10 min, and plasma drug concentrations were determined by the LC-MS/MS method. Measured concentration values below 80% of the lower limit of quantification of the analyzed subject were considered not detected (counted as zero) or below the limit of quantification (counted as ND); values before the peak concentration Cₘₐₓ were counted as zero, and values after the peak concentration were counted as ND. Pharmacokinetics parameters were calculated using Phoenix WinNonlin 8.0 software.

The main pharmacokinetics parameter experiment results are as follows: The primary pharmacokinetics parameters after the administration of different doses of a suspension of the compound of Formula I to SD rats by oral gavage are shown in Table 5 below.

**Table 5**

| | Mean ± Standard Deviation (%CV) | | | |
|---|---|---|---|---|
| Parameter (unit) | Baloxavir marboxil group | T-3 compound group | T-4 compound group | Compound of Formula I group |
| Tₘₐₓ (h) | 1.0000 (0.250, 2.000) | 1.0000 (0.2500, 2.000) | 1.0000 (0.500, 4.000) | 1.0000 (0.500, 2.000) |
| Cₘₐₓ (ng/mL) | 23.1900 ± 7.9729 (34.38%) | 24.3733 ± 3.7324 (15.31%) | 27.3733 ± 3.7324 (13.64%) | 31.7780 ± 2.4334 (7.66%) |
| AUC 0-t (ng·h/mL) | 157.8227 ± 56.5111 (35.81%) | 160.2841 ± 20.6839 (12.90%) | 177.3194 ± 29.0904 (16.41%) | 203.1047 ± 21.4237 (10.55%) |
| AUC 0-∞ (ng·h/mL) | 160.9111 ± 57.8613 (35.96%) | 162.9600 ± 26.3678 (16.18%) | 180.0708 ± 35.3936 (19.66%) | 207.7670 ± 25.0910 (12.08%) |
| t1/2 (h) | 4.2016 ± 0.5595 (13.32%) | 4.9121 ± 1.6806 (34.21%) | 5.4989 ± 1.3053 (23.74%) | 6.7874 ± 1.8973 (27.95%) |

The above experiment results show that the compound of the present application exhibits better pharmacokinetics properties than baloxavir marboxil, the T-3 compound and the T-4 compound.

## Claims

1. A compound which is a compound of Formula X, or a pharmaceutically acceptable salt thereof, 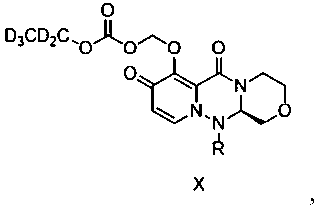 wherein R is selected from H, Fmoc or

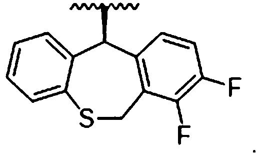

2. A pharmaceutical composition, comprising the compound according to claim 1 or the pharmaceutically acceptable salt thereof and a pharmaceutically acceptable adjuvant, wherein R is

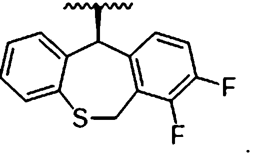

3. The pharmaceutical composition according to claim 2, wherein the pharmaceutical composition further comprises one or more other therapeutic agents.

4. The pharmaceutical composition according to claim 3, wherein the other therapeutic agents are selected from anti-influenza viral agents or vaccines.

5. The pharmaceutical composition according to claim 4, wherein the other therapeutic agents are optionally selected from at least one of oseltamivir, zanamivir, peramivir, laninamivir, favipiravir, Abidol hydrochloride, amantadine, rimantadine, ribavirin, stachyflin, ingavirin, baloxavir or a vaccine.

6. Use of the compound according to claim 1 or the pharmaceutical composition according to any one of claims 2 to 5 in the preparation of a drug for preventing, handling, treating or alleviating an influenza viral infection disease in a patient, wherein R is

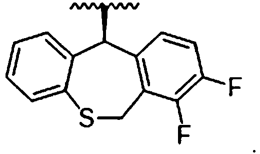

7. A method for preparing a compound of Formula X, wherein R is Fmoc, and the method comprises the following steps:
step one: carrying out an esterification reaction on Compound IV, Compound V, an acid-binding agent and a reaction solvent at a certain temperature to produce a compound of Formula VI; and
step two: carrying out a substitution reaction on Compound III, Compound VI, an alkali, a catalyst and a reaction solvent at a certain temperature to produce a compound of Formula VII;

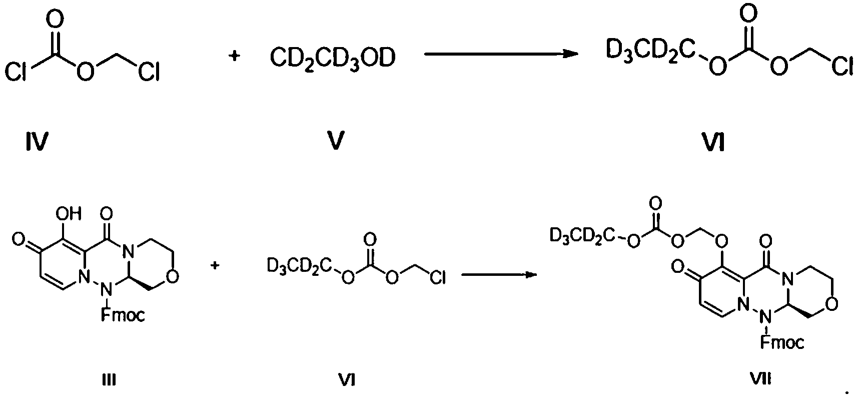

8. A method for preparing a compound of Formula X, wherein R is H, and the method comprises: carrying out a deprotection reaction on Compound VII, an alkali and a reaction solvent at a certain temperature to produce a compound of Formula VIII:

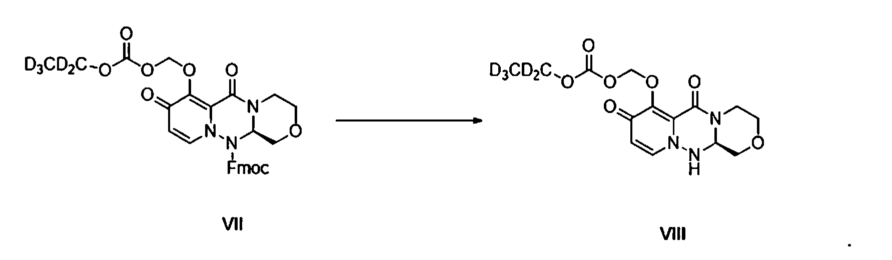

9. A method for preparing a compound of Formula X, wherein R is 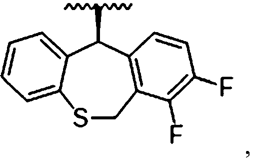 and the method comprises: carrying out a condensation reaction on Compound VIII, Compound IX, a condensing agent, a catalyst and a reaction solvent at a certain temperature to produce a compound of Formula I:

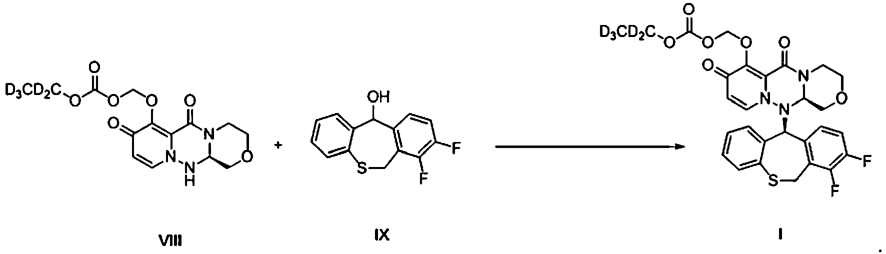

10. A method for preparing a compound of Formula X, wherein R is 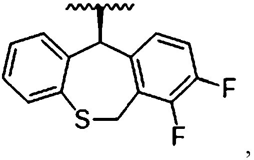 and the method comprises the following steps:
step one: carrying out a condensation reaction on Compound II, 9-fluorenylmethyl chloroformate, sodium bicarbonate and 1,4-dioxane at 0-40 °C, and carrying out a hydrolysis reaction with lithium chloride and *N*-methylpyrrolidone at 60-100 °C to produce a compound of Formula III;
step two: carrying out an esterification reaction on Compound IV, Compound V, pyridine and dichloromethane at -10-40 °C to produce a compound of Formula VI, and carrying out a substitution reaction on Compound III, Compound VI, potassium carbonate, potassium iodide and *N*,*N*-dimethylformamide at 40-70 °C to produce a compound of Formula VII;
step three: carrying out a deprotection reaction on Compound VII, hexahydropyridine and dichloromethane at 10-40 °C to produce a compound of Formula VIII; and
step four: carrying out a condensation reaction on Compound VIII, Compound IX, 1-propyl phosphoric acid cyclic anhydride, methanesulfonic acid, ethyl acetate and cyclohexane at 60-80 °C to produce a compound of Formula I;
